# EUROPÄISCHE PATENTSCHRIFT

(11) **EP 1 565 435 B1**
(45) Veröffentlichungstag und Bekanntmachung des Hinweises auf die Patenterteilung: **05.04.2006**
(21) Anmeldenummer: 03789024.1
(22) Anmeldetag: 11.11.2003
(51) Int. Cl.: C07D 201/16, C07D 201/08

(54) **VERFAHREN ZUR REINIGUNG VON CAPROLACTAM**
METHOD FOR PURIFYING CAPROLACTAM
PROCEDE DE NETTOYAGE DE CAPROLACTAME

(30) Priorität: 13.11.2002 DE 10253094
(43) Veröffentlichungstag der Anmeldung: 24.08.2005
(73) Patentinhaber: BASF Aktiengesellschaft, 67056 Ludwigshafen (DE)
(72) Erfinder: LUYKEN, Hermann, 67069 Ludwigshafen (DE); ANSMANN, Andreas, 69168 Wiesloch (DE); BENISCH, Christoph, 68165 Mannheim (DE); BASSLER, Peter, 68519 Viernheim (DE); FISCHER, Rolf-Hartmuth, 69121 Heidelberg (DE); MAIXNER, Stefan, 68723 Schwetzingen (DE); MELDER, Johann-Peter, 67459 Böhl-Iggelheim (DE)
(86) Internationale Anmeldenummer: PCT/EP2003/012558
(87) Internationale Veröffentlichungsnummer: WO 2004/043915

(56) Entgegenhaltungen:
- WO-A-01/83441
- WO-A-01/83442
- WO-A-01/90065
- WO-A-01/94308
- WO-A-03/045911
- US-A- 5 495 016
- US-A- 5 693 793

## Beschreibung

Die vorliegende Erfindung betrifft ein Verfahren zur Reinigung von Rohcaprolactam, das erhalten wurde, indem man
1. eine Mischung (I) enthaltend 6-Aminocapronitril und Wasser zu einer Mischung (II) enthaltend Caprolactam, Ammoniak, Wasser, Hochsieder und Leichtsieder umsetzt in Gegenwart eines Katalysators, anschließend
2. aus Mischung (II) Ammoniak entfernt unter Erhalt einer Mischung (III) enthaltend Caprolactam, Wasser, Hochsieder und Leichtsieder, anschließend
3. aus Mischung (III) Wasser ganz oder teilweise entfernt unter Erhalt eines Rohcaprolactams (IV) enthaltend Caprolactam, Hochsieder und Leichtsieder,
   dadurch gekennzeichnet, daß man
   a) das Rohcaprolactam und eine anorganische Säure, die einen Siedepunkt oberhalb des Siedepunkts von Caprolactam unter den Destillationsbedingungen gemäß den nachfolgenden Schritten b) bis h) aufweist, einer ersten Destillationsvorrichtung K1 zuführt,
   b) in der ersten Destillationsvorrichtung K1 das Rohcaprolactam und die anorganische Säure destilliert wobei man der Destillationsvorrichtung K1 einen ersten Teilstrom im Sumpfbereich und einen zweiten Teilstrom im Kopfbereich entnimmt,
   c) zweiten Teilstrom aus Schritt b) einer zweiten Destillationsvorrichtung K2 zuführt,
   d) in der zweiten Destillationsvorrichtung K2 den zweiten Teilstroms aus Schritt b) destilliert, wobei man der Destillationsvorrichtung K2 einen ersten Teilstrom im Sumpfbereich und einen zweiten Teilstrom im Kopfbereich entnimmt,
   e) den ersten Teilstrom aus Schritt d) einer dritten Destillationsvorrichtung K3 zuführt,
   f) in der dritten Destillationsvorrichtung K3 den ersten Teilstrom aus Schritt d) destilliert, wobei man der Destillationsvorrichtung K3 einen ersten Teilstrom im Sumpfbereich und gereinigtes Caprolactam im Kopfbereich entnimmt, und
   g) den ersten Teilstrom aus Schritt f) der ersten Destillationsvorrichtung K1 zuführt.

Verfahren zur Herstellung von Caprolactam sind allgemein bekannt.

Es ist ebenfalls, beispielsweise aus Ullmann's Encyclopedia of Industrial Chemistry, 5. Ed., Vol. A5, VCH Verlagsgesellschaft mbH, Weinheim (Deutschland), 1986, Seite 46-48, oder Kirk-Othmer, Encyclopedia of Chemical Technology, 4. Ed., Vol. 4, John Wiley & Sons, New York, 1992, Seite 836, allgemein bekannt, daß Caprolactam, das für die Herstellung von Polymeren verwendet wird, eine Reinheit von 99,9 bis 99,94 % aufweisen muß, wobei die Hauptverunreinigung üblicherweise Wasser in einer Menge von 0,04 bis 0,1 % ist. Andere Verunreinigungen dürfen nur im Bereich von maximal wenigen ppm enthalten sein.
So kann Caprolactam durch Beckmann-Umlagerung von Cyclohexanonoxim mit Schwefelsäure oder Oleum hergestellt werden. Nach Neutralisation des auf diese Weise erhaltenen Gemischs mit Ammoniak kann das Caprolactam von dem als Nebenprodukt enstandenen Ammoniumsulfat durch Extraktion mit einem organischen Lösungsmittel erhalten werden.

In Abhängigkeit von den Verfahren zur Herstellung der zur Darstellung des Cyclohexanonoxims eingesetzten Edukte, wie Cyclohexanon und Hydroxylammoniumsulfat, den Oximierungs- und Umlagerungsbedingungen enthält das rohe Caprolactam, das durch Beckmann-Umlagerung erhalten wurde, Verunreinigungen, die sich in Art und Umfang unterscheiden. Typische Verunreinigungen von rohem Caprolactam, das durch Beckmann-Umlagerung hergestellt wurde, sind C-Methylcaprolactame, 6-Methylvalerolactam und n-Pentylacetamid.

Zur Reinigung des bei der Beckmann-Umlagerung erhaltenen Roh-Caprolactams sind verschiedene Verfahren beschrieben.

Gemäß DE A-1253716 kann das Roh-Caprolactam durch Hydrierung in Suspension in Gegenwart eines Katalysators und unter Zusatz einer Säure gereinigt werden.

Gemäß DE-A-1253716 kann das Roh-Caprolactam durch Hydrierung in Suspension in Gegenwart eines Katalysators und unter Zusatz einer Base gereinigt werden.

DD-A-75083 beschreibt ein Verfahren zur Reinigung von Roh-Caprolactam, in dem das Roh-Caprolactam zunächst destilliert und anschließend, gelöst in einem organischen Lösungsmittel, in Gegenwart eines Katalysators hydriert und dann mit einem lonentauscher behandelt wird.

Gemäß EP-A-411455 können die charakteristischen wichtigen Qualitätsmerkmale für Caprolactam eingehalten werden, indem man das Roh-Caprolactam kontinuierlich in einem Flüssigphasen-Verfahren hydriert.

Roh-Caprolactam, das durch Hydroformylierung von 3-Pentensäure und/oder ihren Estern zu 5-Formylvaleriansäure(estern) als Hauptprodukten und 4- und 3-Formylvaleriansäure(estern) als Nebenprodukten, extraktiver (WO 97/02228) oder destillativer (WO 97/06126) Abtrennung dieser verzweigten Formylvaleriansäure(ester), aminierender Hydrierung von 5-Formylvaleriansäure(estern) zu 6-Aminocapronsäure(estern) und/oder 6-Aminocapronsäureamid und Cyclisierung von 6-Aminocapronsäure(estern) oder 6-Aminocapronsäureamid erhalten wird, enthält andere typische Verunreinigungen.

So ist beispielsweise aus WO 99/48867, Beispiel 1, bekannt, ausgehend von 5-Formylvaleriansäureestem, nach WO 98/37063, Beispiel 9 aus Gemischen aus 6-Aminocapronsäure, 6-Aminocapronsäureamid und entsprechenden Oligomeren erhaltenes Rohcaprolactam unter Zusatz von 10 Gew.-% Wasser, zu kristallisieren. In diesem Rohcaprolactam, aus dem Hoch- und Leichtsieder vor der Kristallisation nicht abgetrennt wurden, waren 6345 ppm N-Methylcaprolactam, 100 ppm 5-Methylvalerolactam, 78 ppm Valeramid und andere Verunreinigungen enthalten. Die Rohcaprotactam/Wasser-Schmelze wurde bei 50°C homogenisiert und dann auf 30°C abgekühlt. Die ausgefallenen Kristalle wurden abfiltriert und 2 bis 3 Mal mit wässrigem Caprolactam gewaschen. 5-Methylvalerolactam und Valeramid wurden auf 1 ppm, N-Methylcaprolactam auf 51 ppm abgereichert. Aus 73,6 g Rohlactam wurden 33,7 g Reinlactam erhalten (Caprolactam-Ausbeute: 45,8 %). Die Kennzahl der flüchtigen Basen (VB) wurde erst durch eine zweite Kristallisation erreicht. Wurden nach WO 99/48867. Beispiel 3, aus dem Rohcaprolactam vor der Kristallisation Hoch- und Leichtsieder abgetrennt, so betrug die Caprolactam-Ausbeute nach der Kristallisation 52 %.

Aus WO 99/65873 ist weiterhin bekannt, Caprolactam aus Gemischen mit 4-Ethyl-2-pyrrolidon, 5-Methyl-2-piperidon, 3-Ethyl-2-pyrrolidon und 3-Methyl-2-Piperidon oder Octahydrophenazin an Adsorptionsmitteln wie aktivierte Aktivkohle, Molekularsieben oder Zeolithen selektiv zu adsorbieren und nach Desorption reines Caprolactam zu erhalten. An diese Caprolactam-Abtrennung kann sich eine Schmelzkristallisation oder eine Kristallisation aus einem Lösungsmittel anschließen.

Es ist weiterhin bekannt, Roh-Caprolactam durch Kristallisation zu reinigen, das ausgehend von 6-Aminocapronitril nach WO 98/37063, Anspruch 8, zunächst mit Wasser zu 6-Aminocapronsäure hydrolysiert wird. Dann werden Wasser und durch Hydrolyse gebildeter Ammoniak abgetrennt, die gebildete 6-Aminocapronsäure wird cyclisiert und das dabei anfallende Roh-Caprolactam nach WO 99/48867 kristallisiert.

Caprolactam kann auch erhalten werden durch Reaktion von 6-Aminocapronitril ("ACN") mit Wasser in der Flüssigphase in der Gegenwart oder Abwesenheit eines Katalysators unter Freisetzung von Ammoniak.

Die bei dieser Reaktion erhaltene Mischung enthält neben Caprolactam, Wasser, Ammoniak, gegebenenfalls weiterem flüssigem Verdünnungsmittel Verunreinigungen mit einem Siedepunkt über dem von Caprolactam ("Hochsieder") und solche mit einem Siedepunkt unter dem von Caprolactam ("Leichtsieder").

Aus US-A-496,941, Beispiel, ist bekannt, daß nach der Abtrennung von Wasser, Lösungsmittel, Ammoniak, Leichtsieder und Hochsieder aus einer Mischung, erhalten bei der Umsetzung von ACN mit Wasser und Lösungsmittel, ein rohes Caprolactam mit einer Reinheit von 99,5 % erhalten wird.

Für ein Roh-Caprolactam, das aus ACN in der Flüssigphase erhalten wurde, sind andere Reinigungsverfahren beschrieben, da sich die Verunreinigungen eines solchen Roh-Caprolactams von denen eines Roh-Caprolactams, das durch andere Verfahren erhalten wurde, wie in US-A-5,496,941 beschrieben, deutlich unterscheiden.

Gemäß US-A-5,496,941 wird ACN in einem ersten Schritt in der Flüssigphase zu Caprolactam umgesetzt, Leichtsieder, Wasser, Ammoniak und gegebenenfalls weitere Lösungsmittel gleichzeitig abgetrennt, Hochsieder abgetrennt unter Erhalt eines Roh-Caprolactams in einer Reinheit von 99,5 %, dieses Roh-Caprolactam in Gegenwart eines Katalysators hydriert, das erhaltene Produkt mit einem sauren lonentauscher oder Schwefelsäure behandelt und das erhaltene Produkt in Gegenwart einer Base destilliert.

Aus WO 96/20923 ist ein Verfahren zur Reinigung von Rohcaprolactam bekannt, das aus der Flüssigphasen-Cyclisierung von 6-Aminocapronitril mit Wasser in Gegenwart eines Lösungsmittels und von heterogenen Katalysatoren stammt. Hierbei wird Rohcaprolactam zunächst hydriert, dann mit sauren Agentien behandelt und zuletzt in Gegenwart von Alkali destilliert.

Nachteilig an diesen beiden Reinigungsverfahren ist, dass drei separate Reaktionsschritte für die Herstellung von Rein-Caprolactam benötigt werden.

Aus DE 100 21 199 A1 und DE 100 21 192 ist bekannt, durch Flüssig- oder Gasphasen-Cyclisierung gewonnenes Caprolactam nach Abtrennung von Ammoniak und Wasser durch Kristallisation zu reinigen.

Die genannten Verfahren zur Reinigung von Roh-Caprolactam, das aus ACN hergestellt wurde, weisen den Nachteil auf, daß sie technisch aufwendig und energieintensiv, insbesondere durch die zahlreichen Trennschritte, sind.

Der vorliegenden Erfindung lag die Aufgabe zugrunde, ein Verfahren zur Verfügung zu stellen, das die Herstellung von Caprolactam, das ausgehend von ACN erhalten wurde, in hoher Reinheit auf technisch einfache und energiesparende Weise ermöglicht.

Demgemäß wurde das eingangs definierte Verfahren gefunden.

In dem erfindungsgemäßen Verfahren setzt man ein Rohcaprolactam ein, das durch Umsetzung von 6-Aminocapronitril mit Wasser gemäß den Schritten 1), 2) und 3) erhalten wurde.

Gemäß Schritt 1) wird eine Mischung (I) enthaltend 6-Aminocapronitril, Wasser und gegebenenfalls flüssiges Verdünnungsmittel zu einer Mischung (II) enthaltend Caprolactam, Ammoniak, Wasser, gegebenenfalls flüssiges Verdünnungsmittel, Hochsieder und Leichtsieder in Gegenwart eines die Umsetzung katalytisch fördemden Feststoffes umgesetzt.

Das für Schritt 1) erforderliche ACN kann, wie aus Ullmann's Encyclopedia of Industrial Chemistry, 5. Ed., Vol. A5, VCH Verlagsgesellschaft mbH, Weinheim (Deutschland), 1986, Seite 46, Fig. 8, allgemein bekannt, aus Adipodinitril erhalten werden.
Besonders in Betracht kommt dabei die partielle katalytische Hydrierung von Adipodinitril in Gegenwart von Ammoniak als Lösungsmittel und beispielsweise als Suspensionskatalysator Rhodium auf Magnesiumoxid (US-A-4,601,859), Raney Nickel (US-A-2,762,835, WO 92/21650), Nickel auf Aluminiumoxid (US-A 2,208,598) oder als Festbettkatalysator Cu-Co-Zn-Spinell (DE-B-954416, US-A 2,257,814) oder Eisen (DE-A-42 35 466) oder ein Verfahren gemäß US-A 2,245,129, US-A-2,301,964, EP-A-150295 oder einem in US-A-5,496,941 beschriebenen Verfahren.

Das für diese Umsetzung erforderliche Adipodinitril wird technisch hergestellt, beispielsweise durch doppelte Hydrocyanierung von Butadien in Gegenwart von Nickel enthaltenden Katalysatoren, und ist kommerziell beispielsweise über die Firma Aldrich-Chemie Gesellschaft mbH & Co. KG, Steinheim, Deutschland verfügbar.

Die Umsetzung von Mischung (I) zu Mischung (II) kann gemäß US-A-4,628,085 in der Gasphase an Kieselgel bei 300°C erfolgen.

Ebenso kann diese Umsetzung gemäß US-A-4,625,023 in der Gasphase an einem Kieselgel-oder Kupfer/Chrom/Barium-Titanoxid-Katalysator durchgeführt werden.

Die Umsetzung von Mischung (I) zu Mischung (II) kann auch beispielsweise gemäß EP-A-659 741, WO 96/22974, DE 19632006, WO 99/47500 oder WO 99/28296 erfolgen.

Vorzugsweise kann die Umsetzung in der Gasphase bei Temperaturen von im allgemeinen 200 bis 550°C, vorzugsweise 250 bis 400°C, durchgeführt werden; der Druck liegt im allgemeinen im Bereich von 0,01 bis 10 bar, vorzugsweise bei Normaldruck, wobei darauf zu achten ist, daß das Reaktionsgemisch unter den angewandten Bedingungen zum überwiegenden Teil gasförmig ist.

Die Katalysatorbelastungen betragen üblicherweise 0,05 bis 2, vorzugsweise 0,1 bis 1,5, insbesondere 0,2 bis 1 kg 6-Aminocapronitril pro Liter Katalysatorvolumen pro Stunde.

Die Umsetzung kann diskontinuierlich, vorzugsweise kontinuierlich durchgeführt werden.

Als Reaktoren kommen vorteilhaft solche in Betracht, wie sie im allgemeinen für Gasphasenreaktionen an bewegten oder stationären Feststoff-Katalysatoren bekannt sind. Vorzugsweise können ein Wirbelbettreaktor, vorzugsweise Festbett-Reaktor, wie ein Horden-Reaktor, insbesondere ein Röhrenreaktor, eingesetzt werden. Es sind auch Kombinationen solcher Reaktoren möglich.

Pro mol ACN werden im allgemeinen 1 bis 50, vorzugsweise 1 bis 10 mol Wasser eingesetzt.

Die Mischung (I) kann auch weitere organische Verbindungen enthalten, die unter den Reaktionsbedingungen gasförmig vorliegen, wie Alkohole, Amine oder aromatische oder aliphatische Kohlenwasserstoffe.

Als katalytisch aktive Verbindungen der Katalysatoren können beispielsweise Siliciumdioxid als pyrogen hergestelltes Siliciumdioxid, als Kieselgel, Kieselgur, Quarz oder Mischungen derselben, Kupferchromit, vorzugsweise Aluminiumoxid, Titanoxid, vorzugsweise Titandioxid, Lanthanphosphate, Lanthanoxide in Betracht wie auch Gemische solcher Verbindungen.

Aluminiumoxid ist in allen Modifikationen, die durch Erhitzen der Vorläuferverbindungen Aluminiumhydroxid (Gibbsit, Böhmit, Pseudo-Böhmit, Bayerit und Diaspor) bei unterschiedlichen Temperaturen erhalten werden können, geeignet. Dazu gehören insbesondere gamma- und alpha-Aluminiumoxid und deren Gemische.

Titandioxid ist amorph und in allen seinen Modifikationen, vorzugsweise Anatas und Rutil, sowie Mischungen solcher Modifikationen geeignet.

Lanthanphosphate sind in ihren verschiedenen Modifikationen, stöchiometrischen Verhältnissen zwischen Lanthan und Phosphateinheit und Kondensationsgraden der Phophateinheiten (Monophosphat, Oligophosphate wie Diphosphate oder Triphosphate, Polyphosphate) einzeln oder im Gemisch geeignet.

Diese Verbindungen können in Form von Pulvern, Gries, Splitt, Strängen oder zu Tabletten gepreßt, verwendet werden. Die Form der Verbindungen richtet sich in der Regel nach den Erfordemissen der jeweiligen Reaktionsführung, wobei in Wirbelbettfahrweise vorteilhaft Pulver oder Gries verwendet wird. Bei der Festbettfahrweise werden üblicherweise Tabletten oder Stränge mit Durchmessern zwischen 1 mm und 6 mm verwendet.

Die Verbindungen können in reiner Form (Gehalt des jeweiligen Verbindungen > 80 Gew-%), als Gemisch der oben genannten Verbindungen, wobei die Summe der oben genannten Verbindungen > 80 Gew.-% betragen soll, oder als Trägerkatalysator, wobei die oben genannten Verbindungen auf einen mechanisch und chemisch stabilen Träger meist mit hoher Oberfläche aufgebracht werden können, verwendet werden.

Die reinen Verbindungen können durch Fällung aus wäßrigen Lösungen hergestellt worden sein, z.B. Titandioxid nach dem Sulfatprozeß oder durch andere Verfahren wie die pyrogene Herstellung von feinen Aluminiumoxid-, Titandioxid- oder Zirkondioxid-Pulvem, die käuflich zu erhalten sind.

Zur Herstellung von Gemischen der verschiedenen Verbindungen stehen mehrere Methoden zur Wahl. Die Verbindungen oder deren Vorläuferverbindungen, die durch Calzinieren in die Oxide umwandelbar sind, können z.B. durch eine gemeinsame Fällung aus Lösung hergestellt werden. Dabei wird im allgemeinen eine sehr gute Verteilung der beiden verwendeten Verbindungen erhalten. Die Verbindungs- oder Vorläufergemische können auch durch eine Fällung der einen Verbindung oder Vorläufers in Gegenwart der als Suspension von fein verteilten Teilchen vorliegenden zweiten Verbindung oder Vorläufers ausgefällt werden. Eine weitere Methode besteht im mechanischen Mischen der Verbindungs- oder Vorläuferpulver, wobei dieses Gemisch als Ausgangsmaterial zur Herstellung von Strängen oder Tabletten Verwendung finden kann.

Zur Herstellung von Trägerkatalysatoren bieten sich im Prinzip alle in der Literatur beschriebenen Methoden an. So können die Verbindungen in Form ihrer Sole durch einfaches Tränken auf dem Träger aufgebracht werden. Durch Trocknen und Calzinieren werden die flüchtigen Bestandteile des Sols üblicherweise aus dem Katalysator entfernt. Solche Sole sind für Titandioxid und Aluminiumoxid käuflich erhältlich.

Eine weitere Möglichkeit zum Aufbringen von Schichten der katalytisch aktiven Verbindungen besteht in der Hydrolyse oder Pyrolyse von organischen oder anorganischen Verbindungen. So kann ein keramischer Träger mit Titandioxid durch Hydrolyse von Titan-Isopropylat oder anderen Ti-Alkoxiden in dünner Schicht belegt werden. Weitere geeignete Verbindungen sind unter anderen TiCl4 und , Aluminiumnitrat. Geeignete Träger sind Pulver, Stränge oder Tabletten der genannten Verbindungen selbst oder anderer stabiler Verbindungen wie Steatit oder Siliciumcarbid. Die verwendeten Träger können zur Verbesserung des Stofftransports makroporös ausgestattet sein.

Die Reaktion kann in Gegenwart eines hinsichtlich der Umsetzung von Mischung (I) zu Mischung (II) inerten Gases, vorzugsweise Argon, insbesondere Stickstoff, durchgeführt werden. Das Volumenverhältnis des inerten Gases zu dem unter den Reaktionsbedingungen gasförmigen ACN kann vorteilhaft bis zu 100 betragen.

Als besonders bevorzugt kommt als Schritt 1) ein Verfahren in Betracht, wie es in US-A-5,646,277 oder US-A-5,739,324 oder FR-A-2029540 beschrieben ist.
Gemäß FR-A-2029540 kann die Umsetzung in Gegenwart von Katalysatoren durchgeführt werden, wobei als Katalysatoren metallisches Zn oder Cu-Pulver oder Oxide, Hydroxide, Halogenide, Cyanide des Rubidiums, Bleis, Quecksilbers oder der Elemente mit einer Ordnungszahl 21 bis 30 oder 39 bis 48 Verwendung finden. Die beschriebenen Katalysatoren werden in diskontinuierlich betriebenen Rührautoklaven als Suspensionskatalysatoren eingesetzt.

Bei diesen Verfahren wird die Umsetzung in flüssiger Phase bei Temperaturen von im allgemeinen 140 bis 320°C, vorzugsweise 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil flüssig ist. Die Verweilzeiten liegen im allgemeinen im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.
Die Umsetzung kann diskontinuierlich, vorzugsweise kontinuierlich durchgeführt werden. Als Reaktor kommt ein Rührkessel, Autoklav, vorzugsweise ein Festbett-Röhrenreaktor, in Betracht. Es sind auch Kombinationen solcher Reaktoren möglich.

Pro mol ACN werden im allgemeinen mindestens 0,1 mol, vorzugsweise 0,5 bis 100 und insbesondere 1 bis 20 mol Wasser eingesetzt.

Vorteilhaft wird das ACN in Form einer 1 bis 50 gew.-%igen, insbesondere 5 bis 50 gew.-%igen, besonders vorzugsweise 5 bis 30 gew.-%igen Lösung in Wasser, wobei dann das Lösungsmittel gleichzeitig Reaktionspartner ist, oder in Gemischen enthaltend Wasser und ein flüssiges Verdünnungsmittel eingesetzt. Als flüssiges Verdünnungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer flüssiger Verdünnungsmittel Anwendung finden. Mischungen aus Wasser und Alkanolen im Gewichtsverhältnis Wasser/Alkanol 1-75/25-99, vorzugsweise 1-50/50-99 haben sich in einigen Fällen als besonders vorteilhaft herausgestellt.

Es ist prinzipiell genauso möglich, ACN als Reaktand und gleichzeitig Lösungsmittel anzuwenden.

Als heterogene Katalysatoren können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid als pyrogen hergestelltes Siliciumdioxid, als Kieselgel, Kieselgur, Quarz oder Mischungen derselben, weiterhin Oxide von Metallen der zweiten bis sechsten Nebengruppe des Periodensystems, wie Titanoxid, amorph, als Anatas oder Rutil, Zirkonoxid, Zinkoxid, Manganoxid oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein:

Vanadiniumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin sind Zeolithe, Phosphate und Heteropolysäuren, sowie saure und alkalische lonenaustauscher wie beispielsweise Nafion als geeignete Katalysatoren zu nennen.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Als besonders bevorzugte Katalysatoren, die unter den oben beschriebenen Reaktionsbedingungen sehr hohe Umsätze, Ausbeuten, Selektivitäten und Standzeiten besitzen, kommen heterogene Katalysatoren auf Basis Titanoxid, Zirkonoxid, Ceroxid und Aluminiumoxid in Betracht. Diese können in Form von Pulvern, Gries, Splitt, Strängen oder zu Tabletten gepreßt, verwendet werden. Die Form der Oxide richtet sich in der Regel nach den Erfordernissen der jeweiligen Reaktionsführung, wobei in Suspension Pulver oder Gries verwendet wird. Bei der Festbettfahrweise werden üblicherweise Tabletten oder Stränge mit Durchmessem zwischen 1 mm und 10 mm verwendet.

Aluminiumoxid ist in allen Modifikationen, die durch Erhitzen der Vorläuferverbindungen Aluminiumhydroxid (Gibbsit, Böhmit, Pseudo-Böhmit, Bayerit und Diaspor) bei unterschiedlichen Temperaturen erhalten werden können, geeignet. Dazu gehören insbesondere gamma- und alpha-Aluminiumoxid und deren Gemische.

Die Oxide können in reiner Form (Gehalt des jeweiligen Oxids > 80 Gew.-%), als Gemisch der oben genannten Oxide, wobei die Summe der oben genannten Oxide > 80 Gew.-% betragen soll, oder als Trägerkatalysator, wobei die oben genannten Oxide auf einen mechanisch und chemisch stabilen Träger meist mit hoher Oberfläche aufgebracht werden können, verwendet werden.

Die reinen Oxide können durch Fällung aus wäßrigen Lösungen hergestellt worden sein, z.B. Titandioxid nach dem Sulfatprozeß oder durch andere Verfahren wie die pyrogene Herstellung von feinen Aluminiumoxid-, Titandioxid- oder Zirkondioxid-Pulvern, die käuflich zu erhalten sind.

Zur Herstellung von Gemischen der verschiedenen Oxide stehen mehrere Methoden zur Wahl. Die Oxide oder deren Vorläuferverbindungen, die durch Calzinieren in die Oxide umwandelbar sind, können z.B. durch eine gemeinsame Fällung aus Lösung hergestellt werden. Dabei wird im allgemeinen eine sehr gute Verteilung der beiden verwendeten Oxide erhalten. Die Oxid- oder Vorläufergemische können auch durch eine Fällung des einen Oxids oder Vorläufers in Gegenwart des als Suspension von fein verteilten Teilchen vorliegenden zweiten Oxids oder Vorläufers ausgefällt werden. Eine weitere Methode besteht im mechanischen Mischen der Oxid- oder Vorläuferpulver, wobei dieses Gemisch als Ausgangsmaterial zur Herstellung von Strängen oder Tabletten Verwendung finden kann.

Zur Herstellung von Trägerkatalysatoren bieten sich im Prinzip alle in der Literatur beschriebenen Methoden an. So können die Oxide in Form ihrer Sole durch einfaches Tränken auf dem Träger aufgebracht werden. Durch Trocknen und Calzinieren werden die flüchtigen Bestandteile des Sols üblicherweise aus dem Katalysator entfernt. Solche Sole sind für Titandioxid, Aluminiumoxid und Zirkondioxid käuflich erhältlich.

Eine weitere Möglichkeit zum Aufbringen von Schichten der aktiven Oxide besteht in der Hydrolyse oder Pyrolyse von organischen oder anorganischen Verbindungen. So kann ein keramischer Träger mit Titandioxid durch Hydrolyse von Titan-Isopropylat oder anderen Ti-Alkoxiden in dünner Schicht belegt werden. Weitere geeignete Verbindungen sind unter anderen TiCl4, Zirkonylchlorid, Aluminiumnitrat und Cernitrat. Geeignete Träger sind Pulver, Stränge oder Tabletten der genannten Oxide selbst oder anderer stabiler Oxide wie Siliciumdioxid. Die verwendeten Träger können zur Verbesserung des Stofftransports makroporös ausgestaltet sein.

Gemäß Schritt 2) wird aus Mischung (II) Ammoniak entfernt unter Erhalt einer Mischung (III) enthaltend Caprolactam, Wasser, gegebenenfalls flüssiges Verdünnungsmittel, Hochsieder und Leichtsieder.

Die Abtrennung des Ammoniaks aus Mischung (II) kann prinzipiell nach an sich für die Stofftrennung bekannten Verfahren, wie Extraktion oder vorzugsweise Destillation, oder eine Kombination solcher Verfahren erfolgen.

Die Destillation kann man vorteilhaft bei Sumpftemperaturen von 60 bis 220°C, insbesondere von 100 bis 220°C durchführen. Dabei stellt man üblicherweise einen Druck, gemessen am Kopf der Destillationsverrichtung von 2 bis 30 bar absolut ein.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Gemäß Schritt 3) werden aus Mischung (III) Wasser ganz oder teilweise und gegebenenfalls flüssige Verdünnungsmittel entfernt unter Erhalt eines Rohcaprolactams (IV) enthaltend Caprolactam, Hochsieder und Leichtsieder.

Wurde in Schritt 1) ein flüssiges Verdünnungsmittel eingesetzt können Wasser und flüssiges Verdünnungsmittel in Schritt 3) gleichzeitig oder das Wasser vor oder nach dem flüssigen Verdünnungsmittel abgetrennt werden.

Die Abtrennung des Wassers und gegebenenfalls des flüssigen Verdünnungsmittels aus Mischung (III) kann prinzipiell nach an sich für die Stofftrennung bekannten Verfahren, wie Extraktion, Kristallisation oder vorzugsweise Destillation, oder eine Kombination solcher Verfahren erfolgen.

Die Destillation kann man vorteilhaft bei Sumpftemperaturen von 50 bis 250°C, insbesondere von 100 bis 230°C durchführen.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Besonders bevorzugt ist eine wärmegekoppelte mehrstufige Abtrennung des Wassers und gegebenenfalls des flüssigen Verdünnungsmittels.

Vor der Zuführung des Rohcaprolactams (IV) in Schritt a) kommt die Abtrennung von Leichtsieder oder Hochsieder, vorteilhaft die Abtrennung nur der Hochsieder, weiterhin vorteilhaft die Abtrennung nur der Leichtsieder, besonders vorteilhaft weder eine Abtrennung von Leichtsieder noch von Hochsieder, insbesondere die Abtrennung der Leichtsieder und der Hochsieder aus dem Rohcaprolactam (IV) in Betracht
Werden Leichtsieder und Hochsieder von dem Rohcaprolactam abgetrennt, so können die Leichtsieder vor, nach oder gemeinsam mit, besonders bevorzugt vor den Hochsiedem abgetrennt werden.

Im Falle einer Abtrennung von Leichtsieder und Hochsieder oder nur Hochsieder oder nur Leichtsieder kann die Abtrennung prinzipiell nach an sich für die Stofftrennung bekannten Verfahren, wie Extraktion, Kristallisation oder vorzugsweise Destillation, oder eine Kombination solcher Verfahren verfolgen.

Die Destillation kann man vorteilhaft bei Sumpftemperaturen von 50 bis 250°C, insbesondere von 100 bis 230°C durchführen. Dabei stellt sich üblicherweise ein Druck, gemessen am Kopf der Destillationsvorrichtung von 1 bis 500, vorzugsweise 5 bis 100 mbar absolut ein.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation zur Abtrennung der Leichtsieder kann man in mehreren, wie 2 oder 3 Kolonnen, vorteilhaft einer einzigen Kolonne durchführen.
Die Destillation zur Abtrennung der Hochsieder kann man in mehreren, wie 2 oder 3 Kolonnen, vorteilhaft einer einzigen Kolonne durchführen.

Als Leichtsieder kommt bei dieser Abtrennung insbesondere 6-Aminocapronitril in Betracht.

Das Rohcaprolactam, das bevorzugt aus dem Kopf der Hochsieder-Abtrennung oder aus dem Sumpf der Leichtsieder-Abtrennung stammt, und eine anorganische Säure, die einen Siedepunkt oberhalb des Siedepunkts von Caprolactam unter den Destillationsbedingungen gemäß den nachfolgenden Schritten b) bis g) aufweist, werden erfindungsgemäß in Schritt a) einer ersten Destillationsvorrichtung K1 zugeführt.

Als anorganische Säure, die einen Siedepunkt oberhalb des Siedepunkts von Caprolactam unter den Destillaüonsbedingungen gemäß den nachfolgenden Schritten b) bis g) aufweist, kommen beispielsweise Schwefelsäure, Phosphorsäure, Borsäure, oder deren saure Salze, wie Alkali-, Erdmetall- oder Erdalkali-Salze oder Gemische solcher Säuren und Salze, vorzugsweise die Säuren oder deren Gemische in Betracht.
In einer vorteilhaften Ausführungsform kann man Phosphorsäure oder deren sauren Salze, wie Alkali-, Erdmetall- oder Erdalkali-Salze oder deren Gemische, insbesondere Phosphorsäure einsetzen. Diese Säuren oder sauren Salze können in reiner Form oder im Gemisch mit einem flüssigen Verdünnungsmittel, wie Wasser, eingesetzt werden.

Rohcaprolactam und anorganische Säure kann man der ersten Destillationsvorrichtung K1 getrennt zuführen oder vorzugsweise vor der Zuführung zur ersten Destillationsvorrichtung K1 mischen und als Gemisch dieser Destillationsvorrichtung zuführen.

Die Menge an anorganischer Säure kann vorteilhaft so eingestellt werden, daß der Gehalt an freier Säure, bestimmbar durch Titration, im Sumpfbereich der ersten Destillationsvorrichtung K1 im Bereich von 0,1 bis 5 Gew.-%, insbesondere 0,5 bis 3 Gew.%, bezogen auf insgesamt in diesem Sumpfbereich vorhandene Mischung, beträgt.

Als erste Destillationsvorrichtung kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Setzt man als erste Destillationsvorrichtung eine Kolonne ein, so kann diese Kolonne vorteilhaft 1 bis 30 theoretische Böden, insbesondere 5 bis 20 theoretische Böden aufweisen.

In einer bevorzugten Ausführungsform kann man Rohcaprolactam (IV) auf den Kopf der Destillationsvorrichtung K1 aufgeben.

Gemäß Schritt b) werden in der ersten Destillationsvorrichtung K1 das Rohcaprolactam und die anorganische Säure destilliert.

Für diese Destillation haben sich Sumpftemperaturen von mindestens 120°C, insbesondere mindestens 140°C als vorteilhaft erwiesen.
Für diese Destillation haben sich Sumpftemperaturen von höchstens 220°C, vorzugsweise höchstens 200°C, insbesondere höchstens 190°C als vorteilhaft erwiesen.
Weiterhin kommt vorzugsweise ein Druck am Kopf der ersten Destillationsvorrichtung K1 von mindestens 10 mbar, insbesondere mindestens 40 mbar in Betracht.
Weiterhin kommt vorzugsweise ein Druck am Kopf der ersten Destillationsvorrichtung K1 von höchstens 120 mbar, insbesondere höchstens 100 mbar in Betracht.

Bei dieser Destillation entnimmt man der Destillationsvorrichtung K1 einen ersten Teilstrom im Sumpfbereich und einen zweiten Teilstrom im Kopfbereich.

Die für die Ausübung des erfindungsgemäßen Verfahrens optimale Größe der beiden Teilströme kann dabei durch wenige einfache Vorversuche leicht ermittelt werden.

Vorteilhaft kann man einen Teil des ersten Teilstroms aus Schritt b) dem Rohcaprolactam in Schritt a) zumischen.

Die für die Ausübung des erfindungsgemäßen Verfahrens optimale Größe des dem Rohcaprolactam zugemischten Anteils an diesem ersten Teilstrom kann dabei durch wenige einfache Vorversuche leicht ermittelt werden.
Nach bisherigen Beobachtungen hat sich ein Gewichtsverhältnis des genannten Anteils des Teilstroms zu Rohcaprolactam von mindestens 0,01 als vorteilhaft erwiesen; weiterhin hat sich ein Gewichtsverhältnis des genannten Anteils des Teilstroms zu Rohcaprolactam von höchstens 0,3 als vorteilhaft erwiesen.

Gemäß Schritt c) führt man den zweiten Teilstrom aus Schritt b) einer zweiten Destillationsvorrichtung K2 zu.

Als zweite Destillationsvorrichtung kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Die Destillation kann man in mehreren, wie 2 oder 3 Kolonnen, vorteilhaft einer einzigen Kolonne durchführen.

Setzt man als zweite Destillationsvorrichtung eine Kolonne ein, so kann diese Kolonne vorteilhaft 3 bis 30 theoretische Böden, insbesondere 5 bis 20 theoretische Böden aufweisen.

Gemäß Schritt d) wird in der zweiten Destillationsvorrichtung K2 der zweite Teilstrom aus Schritt b) destilliert.

Nach bisherigen Beobachtungen können Sumpftemperatur und Druck in einem weiten Bereich gewählt werden, wobei vorteilhaft eine Sumpftemperatur von etwa 200°C und eine Sumpftemperatur von etwa 190°C in Betracht kommen.

Bei dieser Destillation entnimmt man der Destillationsvorrichtung K2 einen ersten Teilstrom im Sumpfbereich und einen zweiten Teilstrom im Kopfbereich.

Die für die Ausübung des erfindungsgemäßen Verfahrens optimale Größe der beiden Teilströme kann dabei durch wenige einfache Vorversuche leicht ermittelt werden.

In einer vorteilhaften Ausführungsform kann man den zweiten Teilstrom aus Schritt d) vollständig oder teilweise, vorzugsweise vollständig zwischen Schritt 3) und Schritt a), insbesondere im Falle einer Abtrennung der Leichtsieder zwischen diesen beiden Schritten vor der Abtrennung der Leichtsieder, zurückführen.

Gemäß Schritt e) führt man den ersten Teilstrom aus Schritt e) einer dritten Destillationsvorrichtung K3 zu.

Als dritte Destillationsvorrichtung kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkofonnen, Packungskolonnen oder Füllkörperkolonnen.

Die Destillation kann man in mehreren, wie 2 oder 3 Kolonnen, vorteilhaft einer einzigen Kolonne durchführen.

Setzt man als dritte Destillationsvorrichtung eine Kolonne ein, so kann diese Kolonne vorteilhaft 3 bis 30 theoretische Böden, insbesondere 5 bis 20 theoretische Böden aufweisen.

Gemäß Schritt f) wird in der dritten Destillationsvorrichtung K3 der zweite Teilstrom aus Schritt d) destilliert.

Nach bisherigen Beobachtungen können Sumpftemperatur und Druck bei dieser Destillation in einem weiten Bereich gewählt werden, wobei vorteilhaft eine Sumpftemperatur von etwa 200°C und eine Sumpftemperatur von etwa 190°C in Betracht kommen.

Bei dieser Destillation entnimmt man der Destillationsvorrichtung K3 einen ersten Teilstrom im Sumpfbereich und gereinigtes Caprolactam in Form eines zweiten Teilstroms im Kopfbereich.

Die für die Ausübung des erfindungsgemäßen Verfahrens optimale Größe der beiden Teilströme kann dabei durch wenige einfache Vorversuche leicht ermittelt werden.
Nach bisherigen Beobachtungen hat sich ein Gewichtsverhältnis des im Kopfbereich entnommenen Teilstroms zu dem im Sumpfbereich entnommenen Teilstrom von mindestens 0,3 als vorteilhaft erwiesen; weiterhin hat sich ein Gewichtsverhältnis des im Kopfbereich entnommenen Teilstroms zu dem im Sumpfbereich entnommenen Teilstrom von höchstens 2,0 als vorteilhaft erwiesen.

Gemäß Schritt g) führt man erfindungsgemäß den ersten Teilstrom aus Schritt f) vollständig oder teilweise, vorzugsweise vollständig, der ersten Destillationsvorrichtung K1 zu. In einer vorteilhaften Ausführungsform kann man diesen Strom auf den Kopf der Destillationsvorrichtung K1 aufgeben.

In einer besonders bevorzugten Ausführungsform kann der Druck in einer, zwei oder drei der Destillationsvorrichtungen K1, K2, K3 mindestens 40 mbar, insbesondere mindestens 100 mbar betragen.

Es war überraschend, daß es erfindungsgemäß gelingt, aus ACN hergestelltes Roh-Caprolactam destillativ, lediglich unter Zugabe von Mineralsäuren, in Caprolactam umzuwandeln, das die für die Faserherstellung erforderliche spezifikationsgerechte hohe Reinheit aufweist. Gemäß US-A-5496941 ist hierfür eine katalytische Hydrierung unter Druck, eine Behandlung mit Säuren und eine Behandlung mit Basen notwendig.

### Beispiel

Aus einem Rohcaprolactam-Strom wurden zunächst Leichtsieder bis auf einen Restgehalt von weniger als 3 ppm ACN abgetrennt. Anschließend wurden Hochsieder abgetrennt und das Caprolactam über Kopf gewonnen.

Aus einem Behälter B1 wurden 85 kg/h des leicht- und hochsiederfreiem Rohcaprolactams in den Kopfbereich einer Kolonne K1 geleitet.
Die Kolonne K1 wurde bei einem Kopfdruck von 50 mbar und einer Sumpftemperatur von 170°C betrieben.
Ober Sumpf der Kolonne K1 wurden 5,2 kg/h abgezogen, wovon 0,2 kg/h ausgeschleust und 5,0 kg/h in den Behälter B1 zurückgeführt wurden.
In den Sumpf der Kolonne K1 wurde soviel wässrige H₃PO₄ zudosiert, dass der Gehalt an freier Säure 1 % betrug.
Der Kopfabzug der Kolonne K1 wurde in eine zweite Kolonne K2 geleitet.
Die Kolonne K2 wurde bei einem Kopfdruck von 50 mbar betrieben.
Über Kopf der Kolonnen K2 wurden 5,0 kg/h abgezogen und zur Aufarbeitung zurückgeführt, bei der Rohcaprolactam gewonnen wurde.
Der Sumpfabzug aus Kolonne K2 wurde in den Sumpfbereich einer Kolonne K3 geleitet.
Die Kolonne K3 wurde bei einem Kopfdruck von 50 mbar betrieben.
Ober Sumpf wurden 100 kg/h abgezogen, die in den Kopfbereich von Kolonne K1 zurückgeführt wurden.

Über Kopf wurden 79,75 kg/h Rein-Caprolactam abgezogen.

Die Kennzahlen des in Rein-Caprolactams wurden wie folgt ermittelt:

| Kennzahl | Meßmethode | Messwert | Spezifikation |
|---|---|---|---|
| E290 | ISO 7059 | 0,024 | < 0,05 |
| Farbzahl | ISO 8112 | 0,36 | < 5 |
| Permanganat-Index | ISO 8660 | 2,51 | < 4 |
| Freie Säuren | acidimetrische Titration einer wässrigen Caproalctam-Lösung gegen Tashiro-Indikator | 0,024 meq/kg | <0,05 meq/kg |
| Flüchtige Basen | ISO 8661 | 0,252 meq/kg | < 0,4 meq/kg |

Die Spezifikation bezieht sich auf kommerziell gehandeltes Caprolactam.

## Patentansprüche

1. Verfahren zur Reinigung von Rohcaprolactam, das erhalten wurde, indem man
1) eine Mischung (I) enthaltend 6-Aminocapronitril und Wasser zu einer Mischung (II) enthaltend Caprolactam, Ammoniak, Wasser, Hochsieder und Leichtsieder umsetzt in Gegenwart eines Katalysators, anschließend
2) aus Mischung (II) Ammoniak entfernt unter Erhalt einer Mischung (III) enthaltend Caprolactam, Wasser, Hochsieder und Leichtsieder, anschließend
3) aus Mischung (III) Wasser ganz oder teilweise entfernt unter Erhalt eines Roh-caprolactams (IV) enthaltend Caprolactam, Hochsieder und Leichtsieder,
**dadurch gekennzeichnet, daß** man
a) das Rohcaprolactam und eine anorganische Säure, die einen Siedepunkt oberhalb des Siedepunkts von Caprolactam unter den Destillationsbedingungen gemäß den nachfolgenden Schritten b) bis h) aufweist, einer ersten Destillationsvorrichtung K1 zuführt,
b) in der ersten Destillationsvorrichtung K1 das Rohcaprolactam und die anorganische Säure destilliert, wobei man der Destillationsvorrichtung K1 einen ersten Teilstrom im Sumpfbereich und einen zweiten Teilstrom im Kopfbereich entnimmt,
c) den zweiten Teilstrom aus Schritt b) einer zweiten Destillationsvorrichtung K2 zuführt,
d) in der zweiten Destillationsvorrichtung K2 den zweiten Teilstroms aus Schritt b) destilliert, wobei man der Destillationsvorrichtung K2 einen ersten Teilstrom im Sumpfbereich und einen zweiten Teilstrom im Kopfbereich entnimmt,
e) den ersten Teilstrom aus Schritt d) einer dritten Destillationsvorrichtung K3 zuführt,
f) in der dritten Destillationsvorrichtung K3 den ersten Teilstrom aus Schritt d) destilliert, wobei man der Destillationsvorrichtung K3 einen ersten Teilstrom im Sumpfbereich und gereinigtes Caprolactam im Kopfbereich entnimmt, und
g) den ersten Teilstrom aus Schritt f) der ersten Destillationsvorrichtung K1 zuführt.

2. Verfahren nach Anspruch 1, wobei Mischung (I) zusätzlich ein organisches flüssiges Verdünnungsmittel enthält.

3. Verfahren nach Anspruch 2, wobei man das flüssige Verdünnungsmittel in Schritt 3) vor, während oder nach der Abtrennung des Wassers aus der Mischung (III) entfernt.

4. Verfahren nach den Ansprüchen 1 bis 3, wobei man zwischen Schritt 3) und Schritt a) Leichtsieder abtrennt oder Hochsieder abtrennt oder Leichtsieder und Hochsieder abtrennt.

5. Verfahren nach Anspruch 4, wobei man zuerst Leichtsieder und anschließend Hochsieder abtrennt.

6. Verfahren nach Anspruch 4 oder 5, wobei man 6-Aminocapronitril als Leichtsieder abtrennt.

7. Verfahren nach den Ansprüchen 1 bis 6, wobei man als anorganische Säure Phosphorsäure einsetzt.

8. Verfahren nach den Ansprüchen 1 bis 7, wobei das Gewichtsverhältnis von dem in Schritt f) im Kopfbereich entnommenen Teilstroms zu dem im Sumpfbereich entnommenen Teilstrom im Bereich von 0,3 und 2,0 liegt.

9. Verfahren nach den Ansprüchen 1 bis 8, wobei man mindestens einen Teil des in Schritt b) erhaltenen ersten Teilstroms dem Rohcaprolactam gemäß Präambel zumischt.

10. Verfahren nach Anspruch 9, wobei das Gewichtsverhältnis des dem Rohcaprolactam zugemischten Teilstroms zu Rohcaprolactam im Bereich von 0,01 bis 0,3 liegt.

11. Verfahren nach den Ansprüchen 1 bis 10, wobei man mindestens einen Teil des in Schritt d) erhaltenen zweiten Teilstroms in Schritt 3) vor Abtrennung der Leichtsieder zurückführt.

12. Verfahren nach den Ansprüchen 1 bis 11, wobei die Sumpftemperatur in der Destillationsvorrichtung K1 im Bereich von 120 bis 200°C liegt.

13. Verfahren nach den Ansprüchen 1 bis 12, wobei der Druck in einer, zwei oder drei der Destillationsvorrichtungen K1, K2, K3 mindestens 40 mbar, gemessen im Kopfbereich/Sumpfbereich ist.

14. Verfahren nach den Ansprüchen 1 bis 13, wobei der erste Teilstrom aus Schritt f) auf den Kopf der Destillationsvorrichtung K1 gegeben wird.

## Claims

1. A process for purifying crude caprolactam which has been obtained by
1) converting a mixture (I) comprising 6-aminocapronitrile and water to a mixture (II) comprising caprolactam, ammonia, water, high boilers and low boilers in the presence of a catalyst, then
2) removing ammonia from mixture (II) to obtain a mixture (III) comprising caprolactam, water, high boilers and low boilers, then
3) completely or partly removing water from mixture (III) to obtain crude caprolactam (IV) comprising caprolactam, high boilers and low boilers,
which comprises
a) feeding the crude caprolactam and an inorganic acid which has a boiling point above the boiling point of caprolactam under the distillation conditions of the following steps b) to h) to a first distillation apparatus C1,
b) distilling the crude caprolactam and the inorganic acid in the first distillation apparatus C1, and removing a first substream in the bottom region and a second substream in the top region of the distillation apparatus C1,
c) feeding the second substream from step b) to a second distillation apparatus C2,
d) distilling the second substream from step b) in the second distillation apparatus C2, and removing a first substream in the bottom region and a second substream in the top region of the distillation apparatus C2,
e) feeding the first substream from step d) to a third distillation apparatus C3,
f) distilling the first substream from d) in the third distillation apparatus C3, and removing a first substream in the bottom region and purified caprolactam in the top region of distillation apparatus C3, and
g) feeding the first substream from step f) to the first distillation apparatus C1.

2. The process according to claim 1, wherein mixture (I) additionally comprises an organic liquid diluent.

3. The process according to claim 2, wherein the liquid diluent is removed in step 3), before, during or after the removal of water from mixture (III).

4. The process according to any of claims 1 to 3, wherein low boilers are removed or high boilers are removed or low boilers and high boilers are removed between step 3) and step a).

5. The process according to claim 4, wherein first low boilers and then high boilers are removed.

6. The process according to claim 4 or 5, wherein 6-aminocapronitrile is removed as a low boiler.

7. The process according to any of claims 1 to 6, wherein the inorganic acid used is phosphoric acid.

8. The process according to any of claims 1 to 7, wherein the weight ratio of the substream removed in the top region to the substream removed in the bottom region in step f) is in the range from 0.3 to 2.0.

9. The process according to any of claims 1 to 8, wherein at least a portion of the first substream obtained in step b) is mixed with the crude caprolactam according to the preamble.

10. The process according to claim 9, wherein the weight ratio of the substream mixed with the crude caprolactam to crude caprolactam is in the range from 0.01 to 0.3.

11. The process according to any of claims 1 to 10, wherein at least a portion of the second substream obtained in step d) is recycled in step 3) before the low boilers are removed.

12. The process according to any of claims 1 to 11, wherein the bottom temperature in the distillation apparatus C1 is in the range from 120 to 200°C.

13. The process according to any of claims 1 to 12, wherein the pressure in one, two or three of distillation apparatus C1, C2, C3 is at least 40 bar, measured in the top region/bottom region.

14. The process according to any of claims 1 to 13, wherein the first substream from step f) is introduced at the top of the distillation apparatus C1.

## Revendications

1. Procédé de nettoyage de caprolactame brut, qui a été obtenu
1) en faisant réagir, en présence d'un catalyseur, un mélange (I) contenant du 6-aminocapronitrile et de l'eau pour former un mélange (II) contenant du caprolactame, de l'ammoniac, de l'eau, des substances à haut point d'ébullition et des substances à bas point d'ébullition, puis
2) en éliminant de l'ammoniac du mélange (II), avec obtention d'un mélange (III) contenant du caprolactame, de l'eau, des substances à haut point d'ébullition et des substances à bas point d'ébullition, puis
3) en éliminant l'eau totalement ou partiellement du mélange (III), avec obtention d'un caprolactame brut (IV) contenant du caprolactame, des substances à haut point d'ébullition et des substances à bas point d'ébullition,
**caractérisé en ce que**
a) on amène à un premier dispositif de distillation K1 le caprolactame brut et un acide inorganique qui présente un point d'ébullition supérieur au point d'ébullition du caprolactame dans les conditions de distillation conformes aux étapes suivantes b) à h),
b) dans le premier dispositif de distillation K1, on distille le caprolactame brut et l'acide inorganique, en prélevant du dispositif de distillation K1 un premier courant partiel dans la zone de fond et un deuxième courant partiel dans la zone de tête,
c) on amène le deuxième courant partiel de l'étape b) à un deuxième dispositif de distillation K2,
d) dans le deuxième dispositif de distillation K2, on distille le deuxième courant partiel de l'étape b), en prélevant du dispositif de distillation K2 un premier courant partiel dans la zone de fond et un deuxième courant partiel dans la zone de tête,
e) on amène le premier courant partiel de l'étape d) à un troisième dispositif de distillation K3,
f) dans le troisième dispositif de distillation K3, on distille le premier courant partiel de l'étape d), en prélevant du dispositif de distillation K3 un premier courant partiel dans la zone de fond et du caprolactame purifié dans la zone de tête, et
g) on amène le premier courant partiel de l'étape f) au premier dispositif de distillation K1.

2. Procédé suivant la revendication 1, dans lequel le mélange (I) contient en supplément un diluant liquide organique.

3. Procédé suivant la revendication 2, dans lequel on élimine le diluant liquide dans l'étape 3) avant, pendant ou après la séparation de l'eau à partir du mélange (III).

4. Procédé suivant les revendications 1 à 3, dans lequel, entre l'étape 3) et l'étape a), on sépare des substances à bas point d'ébullition ou des substances à haut point d'ébullition ou des substances à bas point d'ébullition et des substances à haut point d'ébullition.

5. Procédé suivant la revendication 4, dans lequel on sépare tout d'abord des substances à bas point d'ébullition et ensuite des substances à haut point d'ébullition.

6. Procédé suivant la revendication 4 ou 5, dans lequel on sépare du 6-aminocapronitrile comme substance à bas point d'ébullition.

7. Procédé suivant les revendications 1 à 6, dans lequel on met en oeuvre de l'acide phosphorique comme acide inorganique.

8. Procédé suivant les revendications 1 à 7, dans lequel le rapport pondéral entre le courant partiel prélevé dans la zone de tête dans l'étape f) et le courant partiel prélevé dans la zone de fond est de l'ordre de 0,3 à 2,0.

9. Procédé suivant les revendications 1 à 8, dans lequel on mélange au moins une partie du premier courant partiel obtenu dans l'étape b) au caprolactame brut conforme au préambule.

10. Procédé suivant la revendication 9, dans lequel le rapport pondéral entre le courant partiel mélangé au caprolactame brut et le caprolactame brut est de l'ordre de 0,01 à 0,3.

11. Procédé suivant les revendications 1 à 10, dans lequel, avant la séparation des substances à bas point d'ébullition, on recycle dans l'étape 3) au moins une partie du deuxième courant partiel obtenu dans l'étape d).

12. Procédé suivant les revendications 1 à 11, dans lequel la température de fond dans le dispositif de distillation K1 est de l'ordre de 120 à 200°C.

13. Procédé suivant les revendications 1 à 12, dans lequel la pression dans un, deux ou trois des dispositifs de distillation K1, K2, K3 est au moins de 40 mbar, mesurée dans la zone de tête/zone de fond.

14. Procédé suivant les revendications 1 à 13, dans lequel le premier courant partiel de l'étape f) est fourni à la tête du dispositif de distillation K1.
